# EUROPEAN PATENT APPLICATION

(11) **EP 4 467 151 A1**
(43) Date of publication of application: **27.11.2024**
(21) Application number: 22936351.0
(22) Date of filing: 27.10.2022
(51) Int. Cl.: A61K 36/904, A61K 36/9068, A61P 17/00, A61P 37/08, A61P 17/04, A61P 1/00

(54) **TRADITIONAL CHINESE MEDICINE FORMULA FOR TREATING URTICARIA, ECZEMA, AND ALLERGIC CONSTITUTION**

(30) Priority: 06.04.2022 CN 202210353546
(71) Applicant: Wu, Yakun, Bengbu, Anhui 233000 (CN)
(72) Inventor: Wu, Yakun, Bengbu, Anhui 233000 (CN)
(74) Representative: Porta & Consulenti Associati S.p.A.
(86) International application number: PCT/CN2022/127828
(87) International publication number: WO 2023/193423

(57) **Abstract**

Provided is a traditional Chinese medicine composition for treating urticaria, eczema, and allergic constitution. The composition is a traditional Chinese medicine formula I, a traditional Chinese medicine formula II, or an adjusted combination formula thereof. The traditional Chinese medicine formula I comprises: Scutellariae Radix, Coptidis Rhizoma, Forsythiae fructus, Perillae folium, Magnoliae officinalis cortex, Atractylodis macrocephalae rhizoma, Angelicae Dahuricae radix, Paeoniae alba radix, Cassiae semen, Phellodendri chinensis cortex, Stemonae radix, Artemisiae annuae herba, Anemarrhenae rhizoma, Trichosanthis radix, Glycyrrhizae radix et rhizoma, Menthae haplocalycis herba, Dryopteridis crassirhizomatis rhizoma, Salviae miltiorrhizae radix et rhizoma, Violae herba, and extract of Isatidis folium. The traditional Chinese medicine formula II comprises Glycyrrhizae radix et rhizoma, Menthae haplocalycis herba, Chuanxiong rhizoma, Gentianae radix et rhizoma, Moslae herba, Taraxaci herba, Rhei radix et rhizoma, Senecionis scandentis herba, Zingiberis rhizoma recens, Lonicerae japonicae flos, Chebulae fructus, Belamcandae rhizoma, Pulsatillae radix, Moutan cortex, Magnoliae flos, Corni fructus, Pogostemonis herba, Picrorhizae rhizoma, Dictamni cortex, Schizonepetae herba, and Chrysanthemi flos.

## Description

### TECHNICAL FIELD

Medicine: specifically dermatology/allergy, traditional Chinese medicine/traditional Chinese pharmacology, and microbiology.

### BACKGROUND

The underlying causes of illnesses such as urticaria, eczema, and allergic constitution are yet to be determined by the global medical community. Western medicine focuses on intervening in the process of allergic reactions during the treatment of these conditions, generally using antihistamines, immunomodulatory drugs (such as omalizumab), and hormonal drugs (such as dexamethasone and hydrocortisone). The antihistamines are the most widely used and are effective in controlling the skin symptoms of acute urticaria and acute eczema. However, for patients with allergic constitutions, chronic urticaria, and chronic eczema, the antihistamines can only control the symptoms but not cure them. Moreover, cured patients with acute urticaria and acute eczema cannot eradicate their fundamental triggers of allergies, and there is a risk of acute urticaria and acute eczema transforming into chronic urticaria, chronic eczema, and allergic constitution. Traditional Chinese medicine can treat both the symptoms and root causes of urticaria and eczema. The cured acute urticaria (acute eczema) basically can not be converted into chronic urticaria (chronic eczema). Especially in the treatment of chronic urticaria, chronic eczema, and allergic constitutions, the traditional Chinese medicine shows remarkable effects and needs a long period for drug resistance that can control symptoms for a long period of time, thus exhibiting a potential to cure chronic urticaria, chronic eczema, and allergic constitutions. Unfortunately, the traditional Chinese medicine is far less popular and less frequently used than the antihistamines during the treatment of urticaria, eczema, and allergies. Moreover, traditional Chinese medicine practitioners consider that urticaria, eczema, and allergies as "addiction rash", "wind pathogen" and "wet sore" with vague pathogenesis, making it difficult to cure them with treatments based on the theory of traditional Chinese medicine.

### SUMMARY

From 2010 to September 2015, during the ongoing treatment of the inventor's familial contagious chronic spontaneous severe urticaria (allergic constitution), a medicine for oral administration is used, including: Xueduwan Pills for Skin Diseases from Tongrentang, Yupingfeng Capsules, Runzao Zhiyang Capsules, Baixuan Xiatare Pian Tablets, Bazhen Granules, loratadine tablets, desloratadine dispersible tablets, desloratadine citrate disodium tablets, cetirizine, levocetirizine dihydrochloride tablets, cimetidine tablets, chlorpheniramine, ketotifen, prednisone tablets, and vitamin B complex tablets. Meanwhile, the rash was treated with fluocinolone acetonide ointment, triamcinolone acetonide econazole cream, dexamethasone ointment, paeonol ointment, calamine lotion and other medicines for external use. The above medicine for oral administration is applied in a combined therapy, where a combined therapy with the Xueduwan Pills for Skin Diseases + the desloratadine citrate disodium tablets (or the desloratadine dispersible tablets) + the levocetirizine dihydrochloride tablets, as well as a combined therapy with the Baixuan Xiatare Pian Tablets + the desloratadine citrate disodium tablets (or desloratadine dispersible tablets) + the levocetirizine dihydrochloride tablets have the best efficacy. During this course of treatment, the symptoms of severe chronic spontaneous urticaria (allergic constitution) are alleviated to a certain extent, but are never cured, and an effect of controlling symptoms becomes increasingly worse.

During the continuous treatment of chronic spontaneous urticaria (allergic constitution) over the past 5 years, the inventor found that the Chinese patent medicine "Xueduwan Pills for Skin Diseases from Tongrentang + Baixuan Xiatare Pian Tablets" plays a decisive role in controlling complications such as skin itching, red and itchy eyes, scrotal pruritus, aphtha, ear canal itching, chronic digestive system diseases, and chronic nervous system diseases. Therefore, the inventor began to try Chinese herbal medicine composition treatment in September 2015, and began to use Chinese medicine granule compositions after November 2015. As of November 2018, the types of Chinese herbal medicines (granules) used were more than forty kinds: corn stigma, *Cortex Lycii, Rhizoma Imperatae, Anemarrhenae rhizoma, Rhizoma Smilacis Glabrae,* (bran)-fried *Atractylodis macrocephalae rhizoma, Radix Astragali, Stemonae radix, Cortex Phellodendri, Radix Sophorae Flavescentis, Radix Saposhnikoviae, Schizonepetae herba,* lotus leaf, *Dictamni cortex, Flos Lonicerae* , *Radix Lithospermi, Folium Mori,* (bran)-fried *Semen Coicis, Rhizoma Alismatis, Fructus Amomi Rotundus, Flos Dendranthema morifolium, Fructus Kochiae,* plantain herb, (salt)-processed *Semen Plantaginis, Salviae miltiorrhizae radix et rhizoma,* Paniculate Swallowwort Root, *Moutan cortex, Ramulus Cinnamomi, Radix Codonopsis, Fructus Gardeniae, Radix Paeoniae Rubra, Periostracum Cicadae, Herba Rhodiolae,* stir-fried rice sprout, stir-fried *Endothelium Corneum Gigeriae Galli, Radix Cyathulae,* (raw) *Gypsum Fibrosum,* honeycomb, *Caulis Akebiae, Rhizoma Atractylodis, Radix Rehmanniae, Radix Angelicae Sinensis, Glycyrrhizae radix et rhizoma,* and stir-fried *Fructus Arctii.* Each of the Chinese herbal medicine compositions included 12 to 19 types of the above components. The Chinese herbal medicine compositions could regain control of chronic spontaneous urticaria symptoms and gradually further improve allergic constitution. From April to May 2016, the inventor tried to take the Chinese patent medicine Xunmazhenwan Pills for urticaria orally, and was injected with Liertai injection (thymopentin for injection) once a month to regulate the body's immunity. Compared with the oral administration of traditional Chinese medicine granules, the efficacy of this therapy was not outstanding, and the Xunmazhenwan Pills for urticaria caused great discomfort to the gastrointestinal tract after oral administration. This therapy was discontinued after two months of trial.

After December 2018, the inventor began to try new varieties of traditional Chinese medicine (granules). The original commonly used varieties had become less effective, or received little effect in continuously relieving itching, anti-allergy, and controlling complications such as red and itching eyes, scrotal pruritus, aphtha, ear canal itching, chronic digestive system diseases, and chronic nervous system diseases. As of March 2022, the newly used types of traditional Chinese medicine (granules) included more than fifty types: stir-fried *Semen Armeniacae Amarae,* dried ginger, *Rhizoma Curcumae Longae,* tangerine peel, stir-fried *Bombyx Batryticatus,* (honey)-processed *Herba Ephedrae, Herba Spirodelae, Chuanxiong rhizoma, Scutellariae Radix, Violae herba,* Chinese Honeylocust Spine, *Radix Angelicae* , *Caulis Lonicerae, Radix Angelicae Pubescentis, Forsythiae fructus, Paeoniae alba radix, Gentianae radix et rhizoma,* (ginger)-processed *Magnoliae officinalis cortex,* Flower of Official Magnolia, *Moslae herba, Rhizoma Coptidis, Picrorhizae rhizoma, Trichosanthis radix,* stir-fried *Cassiae semen, Taraxaci herba,* (stir-fried) *Tribulus terrestris, Isatidis folium,* (stir-fried) *Semen brassicae, Radix Platycodi,* (vinegar)-processed *Fructus Schisandrae, Fructus Mume, Radix Bupleuri, Radix Stellariae, Radix Polygoni Multiflori Praeparata, Tetrapanax Papyriferus, Herba Artemisiae Scopariae,* (salt)-processed *Cortex Eucommiae, Rhizoma seu Radix Notopterygii, Chebulae fructus, Herba Houttuyniae, Artemisiae annuae herba, Herba Menthae, Perillae folium, Rhizoma Dryopteris Crassirhizomae, Belamcandae rhizoma, Flos Chrysanthemi Indici, Magnoliae flos, Poria, Caulis Spatholobi, Concha Haliotidis,* snake slough, *Flos Caryophylli, Corni fructus, Zingiberis rhizoma recens, Herba Agastachis,* (cooked) *Rhei radix et rhizoma, Senecionis scandentis herba,* and *Pulsatillae radix.* At the same time, some types of traditional Chinese medicine granules continue to be used: *Glycyrrhizae radix et rhizoma,* (salt)-processed *Anemarrhenae rhizoma, Moutan cortex,* (bran)-fried *Atractylodis macrocephalae rhizoma, Radix Astragali, Radix Sophorae Flavescentis, Schizonepetae herba, Radix Saposhnikoviae, Ramulus Cinnamomi, Dictamni cortex, Folium Mori, Salviae miltiorrhizae radix et rhizoma, Radix Paeoniae Rubra, Stemonae radix, Cortex Phellodendri, Flos Lonicerae,* (stir-fried) *Fructus Arctii, Flos Dendranthema morifolium,* and *Fructus Kochiae.*

After referring to the prescriptions, classic prescriptions, and categories of traditional Chinese medicines that were used or recommended by many Chinese medicine experts in China, a total of nearly a hundred types of Chinese herbal medicines had been selected. After repeated trials and experiments by the inventor, more than thirty types of Chinese herbal medicines with considerable curative effects were selected based on a prescription of 3 to 4 types (approximately equivalent to 90 g to 110 g of decoction pieces), a prescription of 18 to 21 types (approximately equivalent to 180 g to 210 g of decoction pieces), and then a prescription of 26 types (approximately equivalent to 260 g of decoction pieces). 18 to 20 types of them (approximately equivalent to 180 g to 200 g of decoction pieces) were selected to form a Chinese herbal medicine prescription, which has the best curative effect and relatively minimal side effects. As of March 2022, the inventor's familia contagious severe chronic spontaneous urticaria (allergic constitution) had been cured.

Based on more than 10 years of continuous treatment of familial infection, prone to recur when cold, and chronic spontaneous urticaria, the inventor discovered through treatment methods and results that the fundamental causes of allergic constitution, urticaria, and eczema were: microorganisms such as bacteria, fungi, and viruses invade the body. These microorganisms adapt, survive, and multiply in the host body, compete with the host to absorb nutrients, and destroy host tissues, organs, and neurons. At the same time, toxic substances discharged into the host's body fluids through metabolism further harm the host's tissues, organs, and neurons. This continues to cause allergic reactions, severe itching, and various concurrent diseases in the host body.

When one or two parasitic microorganisms are infected and multiply in a short period of time, the host presents with sudden and acute urticaria (or acute eczema). When a variety of infected and parasitic microorganisms invade the host body in different periods of time, adapting, surviving, and gradually multiplying in large numbers, the host body exhibits allergic (allergic) constitution, chronic urticaria, and chronic eczema. Accordingly, the fundamental method to treat allergies, urticaria, and eczema is to resist microorganisms, inhibit their growth and reproduction, kill pathogens, and purify the host's body fluids. Although it is difficult to identify the types of parasitic pathogenic microorganisms, many broad-spectrum antibacterial drugs in traditional Chinese medicine have different effects on inhibiting, resisting, and even killing various types of microorganisms. As a result, traditional Chinese medicine antibacterial prescriptions can be used to treat the disease in a targeted manner. The dispelling wind to eliminate dampness, heat-clearing, and detoxification therapies used by traditional Chinese medicine in the treatment of allergies, chronic urticaria, and chronic eczema have exactly the effect of antibacterial and anti-inflammatory, showing a remarkable curative effect. However, the understanding of allergies in traditional Chinese medicine is limited to the surface of "wind pathogen", "addiction rash" and wet sore", and there is unclear fundamental understanding on a huge number of the microorganisms with a strong mutation ability that cause allergies. Therefore, there is unclear understanding of drug resistance during treatment, and there is no essentially correct treatment concept to guide it. It is difficult to maintain truly effective treatments, making it difficult to cure allergic constitution, chronic urticaria, and chronic eczema.

### Solutions for the problems

Urticaria, eczema, and allergy factors caused by tumors, severe liver and kidney diseases, and thyroid diseases are excluded, and the syndrome is diagnosed as urticaria (regardless of type), allergic constitution, and eczema.

For patients with long-term digestive system and nervous system diseases, such as long-term diarrhea, chronic pharyngitis, aphthous ulcers, chronic (ulcerative) gastroenteritis, anal itching, pruritus vulvae, poor sleep quality, neurasthenia, mental excitation, and physical weakness and other symptoms, a traditional Chinese medicine composition can be applied as follows: 10 g of *Glycyrrhizae radix et rhizoma,* 6 g of *Herba Menthae,* 10 g of *Salviae miltiorrhizae radix et rhizoma,* 10 g of stir-fried *Atractylodis macrocephalae rhizoma,* 10 g of *Chebulae fructus,* 10 g of *Scutellariae Radix,* 6 g of *Coptidis Rhizoma,* 10 g of *Forsythiae fructus,* 10 g of *Perillae folium,* 10 g of *Moslae herba,* 10 g of *Stemonae radix,* 12 g of *Paeoniae alba radix,* 10 g of *Rhizoma Dryopteris Crassirhizomae,* 12 g of salt-processed *Anemarrhenae rhizoma,* 10 g of *Dictamni cortex,* 10 g of *Radix Angelicae* , 10 g of *Taraxaci herba,* 10 g of *Flos Caryophylli,* 10 g of *Corni fructus,* and 15 g of *Violae herba.* Taking this composition can control itching and allergic reactions of the body, relieve diarrhea, relieve mental excitation caused by systemic allergies or itching, and contribute to high-quality sleep at night. Moreover, there are basically no drug resistance within one year of continuous use of the composition.

For patients with long-term respiratory system and nervous system diseases, such as allergic rhinitis, allergic bronchitis/asthma, poor sleep quality, neurasthenia, mental excitation, and physical weakness, drug selection starts from antibacterial, anti-allergic, sedative, and immune system regulation. For example, a composition includes: 10 g of *Glycyrrhizae radix et rhizoma,* 12 g of *Herba Menthae,* 10 g of *Salviae miltiorrhizae radix et rhizoma,* 6 g of *Gentianae radix et rhizoma,* 15 g of *Artemisiae annuae herba,* 10 g of *Scutellariae Radix,* 6 g of *Coptidis Rhizoma,* 10 g of *Forsythiae fructus,* 10 g of *Perillae folium,* 12 g of ginger-processed *Magnoliae officinalis cortex,* 15 g of *Trichosanthis radix,* 15 g of *Senecionis scandentis herba,* 12 g of *Cortex Phellodendri,* 10 g of *Flos Lonicerae* , 15 g of *Isatidis folium,* 6 g of prepared *Rhei radix et rhizoma,* 3 g of *Picrorhizae rhizoma,* 10 g of *Zingiberis rhizoma recens,* and 10 g of *Herba Agastachis.* Taking this composition can control itching and allergic reactions, inhibit allergic rhinitis, allergic bronchitis/asthma, relieve mental excitation caused by systemic allergies or itching, and help to have high-quality sleep at night.

For patients with respiratory system, digestive system, and nervous system diseases, chronic conjunctivitis, chronic pharyngitis, aphthous ulcers, chronic (ulcerative) gastroenteritis, anal itching, vulva itching, allergic rhinitis, allergic bronchitis/asthma, poor sleep quality, neurasthenia, mental excitation, physical weakness, a composition is prepared from the following types of Chinese herbal medicine: 3-9 g of *Scutellariae Radix,* 1.5-8 g of *Coptidis Rhizoma,* 6-15 g of *Forsythiae fructus,* 3-10 g of ginger-processed *Magnoliae officinalis cortex,* 10-30 g of *Violae herba,* 3-15 g of stir-fried *Atractylodis macrocephalae rhizoma,* 3-10 g of *Radix Angelicae* , 5-12 g (15-30 g) of *Paeoniae alba radix,* 6-12 g of *Anemarrhenae rhizoma,* 9-15 g of *Trichosanthis radix,* 10 g of *Glycyrrhizae radix et rhizoma,* 6-30 g of stir-fried *Cassiae semen,* 3-10 g of *Stemonae radix,* 5-10 g of *Perillae folium,* 3-9 g of *Cortex Phellodendri,* 6-15 g (20-40 g) of *Artemisiae annuae herba,* 10-15 g of *Isatidis folium,* 5-15 g of *Rhizoma Dryopteris Crassirhizomae,* 5-15 g of *Salviae miltiorrhizae radix et rhizoma,* 3-6 g of *Herba Menthae,* 15-30 g of *Senecionis scandentis herba,* 15-30 g of *Pulsatillae radix,* 3-9 g of *Moslae herba,* 10-30 g (60 g) of *Taraxaci herba,* 10-20 g of *Flos Lonicerae* , 5-10 g of *Belamcandae rhizoma,* 6-9 g of *Moutan cortex,* 3-10 g of *Magnoliae flos,* 3-12 g of *Rhei radix et rhizoma,* 3-10 g of *Zingiberis rhizoma recens,* 3-6 g of *Chebulae fructus,* 6-15 g of *Dictamni cortex,* 5-10 g of *Cornifructus,* 10-15 g of *Flos Dendranthema morifolium,* 3-10 g of *Chuanxiong rhizoma,* 3-6 g of *Gentianae radix et rhizoma,* 3-10 g of *Schizonepetae herba,* 6-12 g of *Picrorhizae rhizoma,* and 6-10 g of *Herba Agastachis.*

For acute urticaria and sudden severe allergies such as anaphylactic shock, laryngeal edema, hypertension, hypotension, fainting and other symptoms caused by allergies, the allergic symptoms should be controlled, and then the allergens should be eradicated using traditional Chinese medicine compositions, including: second-generation antihistamines combined with traditional Chinese medicine; or systemic, staged combination of glucocorticoid + second-generation antihistamines + traditional Chinese medicine; or systemic and staged combination of epinephrine + second-generation antihistamines + traditional Chinese medicine.

### DETAILED DESCRIPTION OF THE PREFERRED EMBODIMENTS

Components of traditional Chinese medicine composition I is provided, including the following components: scientific extracts from 20 Chinese herbal medicines of *Scutellariae Radix, Coptidis Rhizoma, Forsythiae fructus, Perillae folium, Magnoliae officinalis cortex, Atractylodis macrocephalae rhizoma, Radix Angelicae* , *Paeoniae alba radix, Cassiae semen, Cortex Phellodendri, Stemonae radix, Artemisiae annuae herba, Anemarrhenae rhizoma, Trichosanthis radix, Glycyrrhizae radix et rhizoma, Herba Menthae, Rhizoma Dryopteris Crassirhizomae, Salviae miltiorrhizae radix et rhizoma, Violae herba,* and *Isatidis folium*; where the contents of each component are: 9 g to 10 g of the *Scutellariae Radix,* 6 g to 8 g of the *Coptidis Rhizoma,* 10 g to 12 g of the *Forsythiae fructus,* 10 g of the *Perillae folium,* 10 g to 12 g of ginger-processed *Magnoliae officinalis cortex,* 10 g of stir-fried *Atractylodis macrocephalae rhizoma,* 10 g to 12 g of the *Radix Angelicae* , 10 g to 12 g of the *Paeoniae alba radix,* 10 g to 15 g of stir-fried *Cassiae semen,* 9 g to 12 g of the *Cortex Phellodendri,* 10 g of the *Stemonae radix,* 10 g to 12 g of the *Artemisiae annuae herba,* 10 g to 12 g of salt-processed *Anemarrhenae rhizoma,* 10 g to 12 g of the *Trichosanthis radix,* 9 g to 10 g of the *Glycyrrhizae radix et rhizoma,* 6 g to 12 g of the *Herba Menthae,* 10 g to 12 g of the *Rhizoma Dryopteris Crassirhizomae,* 8 g to 10 g of the *Salviae miltiorrhizae radix et rhizoma,* 10 g to 15 g of the *Violae herba,* and 10 g to 15 g of the *Isatidis folium*; and the traditional Chinese medicine composition I is made using the scientific extracts of the 20 components, and then prepared into a medicine for oral administration comprising an oral pill, a powder, and a capsule; and the scientific extracts of the 20 Chinese herbal medicines are prepared into a medicine for external application and topical application comprising a medicine liquid for external application, an ointment, or a patch that is used for external application and topical application in conjunction with the medicine for oral administration in areas with severe itching of the skin.

Being taken internally + applied externally, this composition can quickly control various types of urticaria, various types of eczema, allergic reaction symptoms and many unexplained severe itching skin conditions. Continuous medication can completely cure the above symptoms. If urticaria, eczema, allergies, and skin itching worsen after taking this composition orally in the early stage, a combination with oral antihistamines can be used to achieve treating both the symptoms and the root cause.

### DETAILED DESCRIPTION OF THE EMBODIMENTS

A traditional Chinese medicine composition II is provided, including the following components: scientifically extracts active ingredients from 21 Chinese herbal medicines of *Glycyrrhizae radix et rhizoma, Herba Menthae, Rhei radix et rhizoma, Moslae herba, Taraxaci herba, Senecionis scandentis herba, Pulsatillae radix, Pulsatillae radix, Magnoliae flos, Belamcandae rhizoma, Chuanxiong rhizoma, Flos Lonicerae* , *Chebulae fructus, Schizonepetae herba, Zingiberis rhizoma recens, Moutan cortex, Dictamni cortex, Flos Dendranthema morifolium, Gentianae radix et rhizoma, Picrorhizae rhizoma, Herba Agastachis,* and *Corni fructus*; where the 21 Chinese herbal medicines have the following contents: 9 g to 10 g of the *Glycyrrhizae radix et rhizoma,* 6 g to 12 g of the *Herba Menthae,* 15 g to 20 g of the *Senecionis scandentis herba,* 15 g to 20 g of the *Pulsatillae radix,* 9 g to 10 g of the *Moslae herba,* 10 g to 15 g of the *Taraxaci herba,* 10 g to 15 g of the *Flos Lonicerae* , 9 g to 10 g of the *Belamcandae rhizoma,* 9 g to 10 g of the *Moutan cortex,* 9 g to 10 g of the *Magnoliae flos,* 5 g to 6 g of prepared *Rhei radix et rhizoma,* 9 g to 10 g of the *Zingiberis rhizoma recens,* 6 g to 10 g of the *Chebulae fructus,* 10 g to 12 g of the *Dictamni cortex,* 9 g to 10 g of the *Corni fructus,* 10 g of *Flos Caryophylli,* 10 g to 12 g of the *Flos Dendranthema morifolium,* 6 g to 10 g of the *Chuanxiong rhizoma,* 5 g to 6 g of the *Gentianae radix et rhizoma,* 9 g to 10 g of the *Schizonepetae herba,* 3 g of the *Picrorhizae rhizoma,* and 9 g to 10 g of the *Herba Agastachis*; and the traditional Chinese medicine composition II is made using the scientific extracts of the active ingredients of the components, and then prepared into a medicine for oral administration comprising an oral pill and a capsule; and the scientific extracts of the Chinese herbal medicines are prepared into a medicine for external application comprising a medicine liquid for external application, an ointment that is used for external application in conjunction with the medicine for oral administration in areas with severe itching of the skin.

Being taken internally + applied externally, this composition can quickly control various types of urticaria, various types of eczema, allergic reaction symptoms and many unexplained severe itching skin conditions. Continuous medication can completely cure these symptoms. If urticaria, eczema, allergies, and skin itching worsen after taking this composition orally in the early stage, a combination with oral antihistamines can be used to achieve treating both the symptoms and the root cause.

### Industrial applicability

The invention treats various types of acute and chronic urticaria, acute and chronic eczema, allergic constitutions, and also treats allergic conjunctivitis, chronic pharyngitis, allergic rhinitis/sinusitis, allergic bronchitis/asthma, oral malignant boils and pyogenic infections, sticky and thick tongue coating, severe bad breath, chronic (ulcerative) gastroenteritis, colitis, proctitis, concurrent joint pain and muscle pain, physical weakness, concurrent ear canal itching, pruritus vulvae, scrotal pruritus, anal pain and itching, hot eyes and itchy eyes; the traditional Chinese medicine composition further treats complications of poor sleep quality, biological clock/endocrine disorders, cranial neurasthenia, complications of listlessness, hyperactivity, and alternating occurrence of the listlessness and the mental excitation, influenza virus, scabies mites, *Helicobacter pylori, Mycoplasma pneumoniae, Shigella dysenteriae,* various types of ringworm and other microbial infections.

### Example

### Patient 1: A 39-year-old male with familial contagious (hereditary) chronic spontaneous urticaria

The patient's grandfather had severe urticaria that caused severe itching all over his body, and even used fire to relieve the itching on his legs when the urticaria broke out. The patient had been suffering from severe scrotal pruritus and skin itching since childhood. When being 7 to 10 years old, the patient would have a large area of red rash on the knee pits, armpits of both shoulders every year, with severe itching. Later, the rash on the knee pits and armpits no longer recurred, and erythema appeared from time to time and in different parts of the body. The outbreaks were particularly frequent and severe on the back vest, waist, inner thighs, and scrotum. As age increased, erythema and severe itching became increasingly severe, and the patient began to suffer from chronic enteritis: ulcerative proctitis, colitis, perennial diarrhea, and repeated colds all year round, which were difficult to cure. At the same time, the patient was accompanied by a series of problems such as severe sleep quality problems, endocrine disorders, cranial neurasthenia, alternating periods of mental excitation and depression, chronic conjunctivitis, chronic pharyngitis, ear canal itching, and allergic rhinitis.

From 2010 to September 2015, the patient had taken medicine for oral administration including: Xueduwan Pills for Skin Diseases from Tongrentang, Yupingfeng Capsules, Runzao Zhiyang Capsules, Baixuan Xiatare Pian Tablets, Bazhen Granules, loratadine tablets, desloratadine dispersible tablets, desloratadine citrate disodium tablets, cetirizine, levocetirizine dihydrochloride tablets, cimetidine tablets, chlorpheniramine, ketotifen, prednisone tablets, and vitamin B complex tablets. Meanwhile, the rash was treated with fluocinolone acetonide ointment, triamcinolone acetonide econazole cream, dexamethasone ointment, paeonol ointment, calamine lotion and other medicines for external application. The medicine for oral administration was in combination with therapy, such as: the combination of desloratadine citrate disodium tablets + levocetirizine tablets + Runzao Zhiyang Capsules; the combination of desloratadine citrate disodium tablets + Xueduwan Pills for Skin Diseases. During the 5-6 years of treatment with this therapy, the initial control effect was relatively desirable, and the symptoms of severe chronic spontaneous urticaria (allergic constitution) were alleviated to a certain extent. However, the patient had never been able to completely control skin itching and other complications, nor could he cure chronic urticaria, and an effect of controlling symptoms was getting increasingly worse. During this treatment process, it was found that the Chinese patent medicines Xueduwan Pills for Skin Diseases from Tongrentang and Baixuan Xiatare Pian Tablets played a decisive role in controlling the symptoms of urticaria and improving allergic constitution.

From November 2015 to May 2022, the patient's severe chronic spontaneous urticaria was treated with traditional Chinese medicine granule composition. After referring to the prescriptions, classic prescriptions, and categories of traditional Chinese medicines that were used or recommended by many Chinese medicine experts in China, a total of nearly a hundred types of Chinese herbal medicines had been selected. After repeated trials and experiments by the inventor, more than thirty types of Chinese herbal medicines with considerable curative effects were selected based on a prescription of 3 to 4 types (approximately equivalent to 90 g to 110 g of decoction pieces), a prescription of 18 to 21 types (approximately equivalent to 180 g to 210 g of decoction pieces), and then a prescription of 26 types (approximately equivalent to 260 g of decoction pieces). These composes the traditional Chinese medicine composition I and the traditional Chinese medicine composition II with the best curative effect and relatively minimal side effects. After using traditional Chinese medicine composition I and traditional Chinese medicine composition II (or cross-exchanging some ingredients of the two compositions), starting from January 2021, after more than 1 year of continuous treatment, the patient's whole body skin erythema and rashes all subsided. Except for the severe rash and itching that suddenly occurred in the knee pits of the patient in February 2022 before recovery (a rebound before recovery, a symptom that had not occurred again in more than 20 years), there was no more itching in the whole body. Along with the disappearance of skin symptoms, the patient's concurrent chronic enteritis: ulcerative proctitis, colitis, hemorrhoids, sleep quality problems, endocrine disorders, persistent and alternating between listlessness and mental excitation, habitual pain in muscles and joints throughout the body, sub-healthy recurring colds, chronic pharyngitis, chronic conjunctivitis, allergic rhinitis, ear canal itching and other diseases related to allergies had all disappeared or are disappearing, and the allergic constitution problem was close to being eliminated.

The typical skin rash healing process was shown in Patient 1 in the drawings of the specification.

### Patient 2: A 54-year-old female with familial contagious (hereditary) chronic spontaneous urticaria

Patient 1's aunt had suffered from severe itching all over her body since she was a child, lasting for more than fifty years. The symptoms of urticaria had been present all year round, and deep red scratches appeared on the scratched areas when itching, and this severe itching was difficult to relieve. The skin returned to normal after the itching, and symptoms worsened in autumn and winter. The patient's diagnosis was familial contagious severe spontaneous cold urticaria.

In February 2014, this patient began to take the Chinese patent medicine Xueduwan Pills for Skin Diseases from Tongrentang, which was occasionally combined with levocetirizine dihydrochloride tablets + desloratadine dispersible tablets to control allergic symptoms. The course of treatment lasted for more than half a year, and the effect was remarkable. The symptoms of skin itching were greatly improved, but were not eradicated. In February 2021, after the recurrence of urticaria, the patient took two commonly used traditional Chinese medicine compositions for Patient 1: traditional Chinese medicine composition I and traditional Chinese medicine composition II (granule-modified formula). Among them, *Scutellariae Radix, Forsythiae fructus,* and *Rhizoma Dryopteris Crassirhizomae* are removed from composition I; *Senecionis scandentis herba, Pulsatillae radix, Magnoliae flos, Belamcandae rhizoma, Flos Caryophylli, Herba Agastachis, Corni fructus, Gentianae radix et rhizoma* are removed from and *Scutellariae Radix, Forsythiae fructus, Ramulus Cinnamomi, Radix Sophorae Flavescentis,* and stir-fried *Fructus Arctii* are added to the composition II. The two groups of composition took 5 doses each, 1 dose per day, fully dissolved in 300 mL of warm water, and half dose was applied in the morning and evening. Each composition could control the symptoms of urticaria when taken internally. After taking 10 doses orally, the recurring urticaria symptoms completely disappeared. During a follow-up visit 5 d after stopping the medication, the urticaria symptoms did not reappear. 10 days after stopping the medication, the weather suddenly turned cold and heavy snow fell at the patient's location. During a follow-up visit, the patient's urticaria symptoms no longer appeared, including no itching while sleeping at night. 20 days after stopping the medication, the patient's urticaria symptoms did not reappear during a follow-up visit. It was initially determined that the patient's urticaria was basically cured.

### Patient 3: A 47-year-old male with chronic spontaneous urticaria

Starting in July and August 2021, the patient suffered from severe urticaria without obvious triggers, erythema all over the body, rash on the palms, some erythema appeared over a large area, and severe itching. These symptoms were also accompanied by chronic gastrointestinal diseases, such as stomach distension and blockage with intermittent severe pain, abdominal pain accompanied by diarrhea, scrotal pruritus, anal itching and pain, internal and external hemorrhoids, and pustules around the mouth. The patient's skin itched severely at night, resulting in poor sleep quality and loss of appetite. Starting from October 2021, this patient went to The Third the People's Hospital of Bengbu for treatment. "Acute urticaria" was diagnosed in the outpatient clinic, and 80-120 mg of methylprednisolone sodium succinate (40 mg/tube) was given intravenously for anti-inflammation and desloratadine and ebastine tablets were taken orally for anti-allergy, while mosapride citrate tablets and rabeprazole sodium enteric-coated tablets were taken orally for gastric protection. Since the disease could not be controlled, the patient was hospitalized on October 10, 2021. During hospitalization, he was given intravenous infusion of methylprednisolone sodium succinate (40 mg/tube) 40 mg once a day for anti-inflammation and vitamin C (1 g/tube) 3 g once a day, calcium gluconate (10 mL: 1 g), cimetidine injection (2 mL: 0.2 g/tube) 200 mg once a day. At the same time, desloratadine citrate disodium tablets, ebastine tablets, and ketotifen fumarate tablets were taken orally for anti-allergy, and mosapride citrate tablets and rabeprazole sodium enteric-coated tablets were taken orally for gastric protection. The skin rash was treated with paeonol ointment and triamcinolone acetonide and econazole cream. By the time he was discharged from the hospital on October 28, the severe itching all over the patient's body was relieved, and the widespread erythema on his trunk and limbs was significantly reduced compared with when he was admitted. However, the itching all over his body was still obvious, and his sleep quality and appetite were still highly poor. The patient still required daily intravenous infusion of methylprednisolone sodium succinate, vitamin C for anti-inflammation, as well as oral administration of desloratadine citrate disodium tablets and ebastine tablets for anti-allergy, and oral administration of mosapride citrate tablets or rabeprazole sodium enteric-coated tablets for gastric protection, thus relieving intermittent severe pain in the stomach and anus.

Starting from January 19, 2022, the patient began to take traditional Chinese medicine compositions orally one dose per day, on the basis of intravenous infusion of methylprednisolone sodium succinate (40 mg/tube) 40 mg once a day for anti-inflammation, vitamin C (1 g/tube) 3 g once a day, and oral antihistamines and gastroprotective drugs. The antihistamines such as desloratadine citrate disodium tablets, ebastine tablets, olopatadine hydrochloride tablets, and ranitidine hydrochloride capsules were successively used; the stomach-protecting drugs such as mosapride citrate tablets, rabeprazole sodium enteric-coated tablets, and omeprazole magnesium enteric-coated tablets were successively used; the traditional Chinese medicine compositions such as the traditional Chinese medicine composition I, the traditional Chinese medicine composition II, and a mixed and adjusted formula of the traditional Chinese medicine composition I and the traditional Chinese medicine composition II were successively used. The intravenous injection was stopped on January 29, 2022, and the hormones prednisone tablets and dexamethasone tablets were added successively for anti-inflammation. On February 15, oral administration of hormones was stopped, and the patient only took oral administration of a combination of traditional Chinese medicine composition containing antibacterial agents, olopatadine hydrochloride tablets for antihistamines, and omeprazole magnesium enteric-coated tablets for gastric protection. On February 26, the oral administration of gastric-protecting drugs was stopped, and the oral administration of olopatadine hydrochloride tablets was reduced (from 1 tablet/day each in the morning and evening to 1 tablet/day), and only the traditional Chinese medicine composition I was taken orally. On March 1, the patient's skin all over his body returned to a healthy state without leaving any marks. The feeling of bloating and blockage in the stomach disappeared, the pain completely disappeared, and he recovered. The patient still insisted on taking the traditional Chinese medicine composition I orally, combined with antihistamine olopatadine hydrochloride 1 tablet/day to control occasional mild itching of the whole body and accompanying anal itching while consolidating the treatment results. Since the patient's chronic spontaneous urticaria was treated with Western medicine, while oral administration of the traditional Chinese medicine composition I (or composition II, or a modified composition thereof) was added, the patient's symptoms such as stomach bloating and blockage, stomach and anal pain, anal itching, body erythema, palm rash, and body itching were significantly reduced every day, and the quality of his sleep at night improved step by step. Although the symptoms recurred during the period, the overall progress was obviously towards recovery, and the symptom control effect was much better than that using Western medicine alone.

The typical skin rash healing process was shown in Patient 3 in the drawings of the specification.

### Patient 4: A 60-year-old female with familial contagious (hereditary) chronic spontaneous urticaria

The patient reported that his mother had suffered from chronic spontaneous urticaria: allergy on the arms, angioedema of the skin with severe itching. The patient had suffered from skin allergies since childhood. The patient had widespread skin angioedema, erythema, rash, and severe itching on the center of back, waists, abdomen, neck, and limbs; the edema varied in size, and some were connected into sheets, showing skin-colored, bright red, or light red; erythema and rash were dark red, most protruded from the skin, and some were connected into sheets; angioedema was common, and most rashes protruded from the skin. Complications included chronic conjunctivitis, ear canal itching, anal itching, and pruritus vulvae, and the eyeballs and inner eyelids were generally blood red, and itching in the eyes was common all year round. The patient had been taking antihistamines such as levocetirizine dihydrochloride and chlorpheniramine maleate for a long time, but the efficacy was poor, and the symptoms did not improve after recurrence. The patient also used Chinese patent medicine Jinchan Zhiyang Capsules and Chinese herbal medicine decoction (other prescriptions), but they were unable to effectively control severe itching all over the body.

On April 8, 2022, the patient began to take a mixed adjusted prescription of the traditional Chinese medicine composition I and traditional Chinese medicine composition II: 10 g of *Artemisiae annuae herba,* 6 g of *Cortex Phellodendri,* 10 g of *Scutellariae Radix,* 10 g of *Forsythiae fructus,* 10 g of *Perillae folium,* 6 g of *Coptidis Rhizoma,* 10 g of salt-processed *Anemarrhenae rhizoma,* 9 g of *Glycyrrhizae radix et rhizoma,* 9 g of *Stemonae radix,* 15 g of *Violae herba,* 6 g of *Herba Menthae,* 10 g of *Taraxaci herba,* 12 g of *Radix Angelicae* , 12 g of ginger-processed *Magnoliae officinalis cortex,* 5 g of *Isatidis folium,* 10 g of *Moslae herba,* 5 g of *Salviae miltiorrhizae radix et rhizoma,* and 9 g of *Zingiberis rhizoma recens,* a total of 7 doses with 18 components. After taking the medicine, skin angioedema was significantly reduced, itching was simultaneously alleviated, erythema and rash were significantly reduced, and sleep quality was improved. The patient requested to continue taking the Chinese medicine granule composition, and then the patient was given a dialectical prescription of a mixed adjusted prescription of the traditional Chinese medicine compositions I and II, ranging from 19 to 21 components. After taking the medicine until late July 2022, the symptoms of chronic urticaria such as frequent severe skin angioedema, severe skin itching, pruritus vulvae, eye redness and itching, and these complications began to be significantly relieved. In August, the patient's body rash and itching symptoms were very mild. From August 30 to September, due to climate change and cooler weather, the patient's chronic spontaneous urticaria worsened again, but the aggravated skin angioedema and itching appeared for a short period of time, generally taking less than 20 min from onset to subsidence. Symptoms rarely reached a serious state, let alone severe skin angioedema and severe itching. After entering October, the patient no longer had angioedema all over the body, and the occasional itching generally did not last long, and a series of symptoms such as ear canal itching, chronic conjunctivitis, pruritus vulvae, and anal itching symptoms that were complications of chronic urticaria gradually became milder and disappear. The patient continued to adhere to the oral administration of traditional Chinese medicine granule composition I, composition II, and the mixed adjusted prescription of compositions I and II to further eradicate the pathogens of allergic constitution and consolidate the previous curative effect.

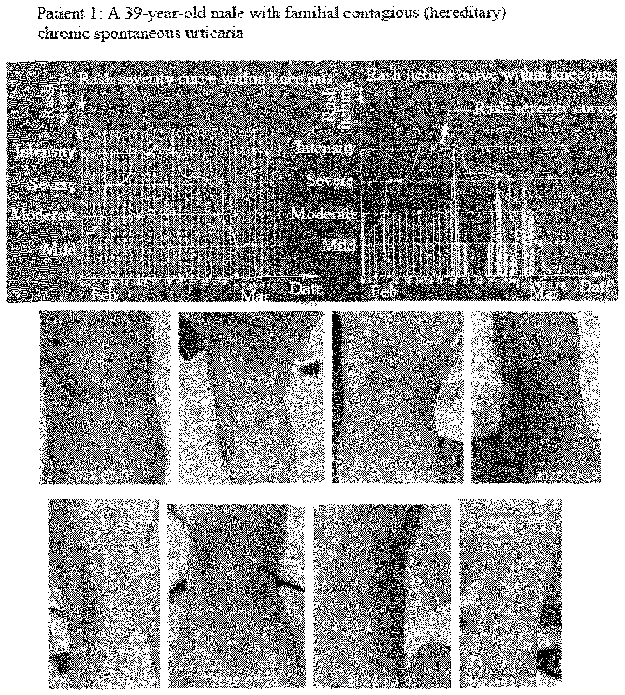

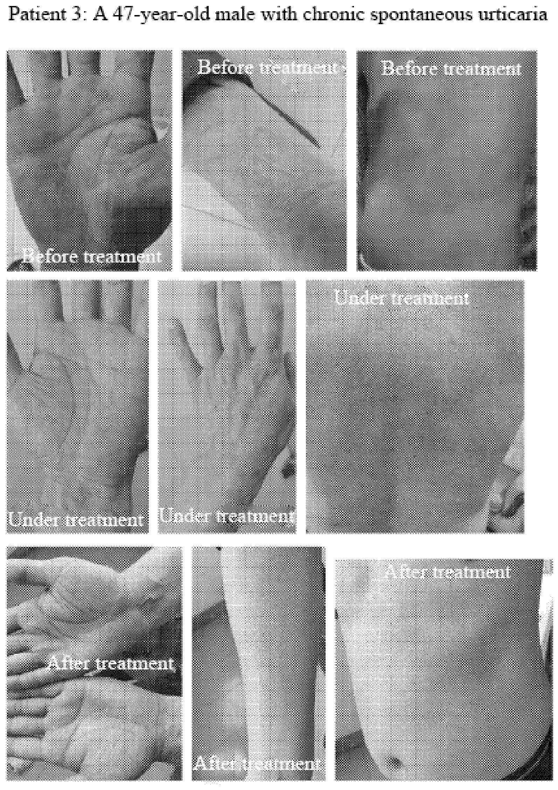

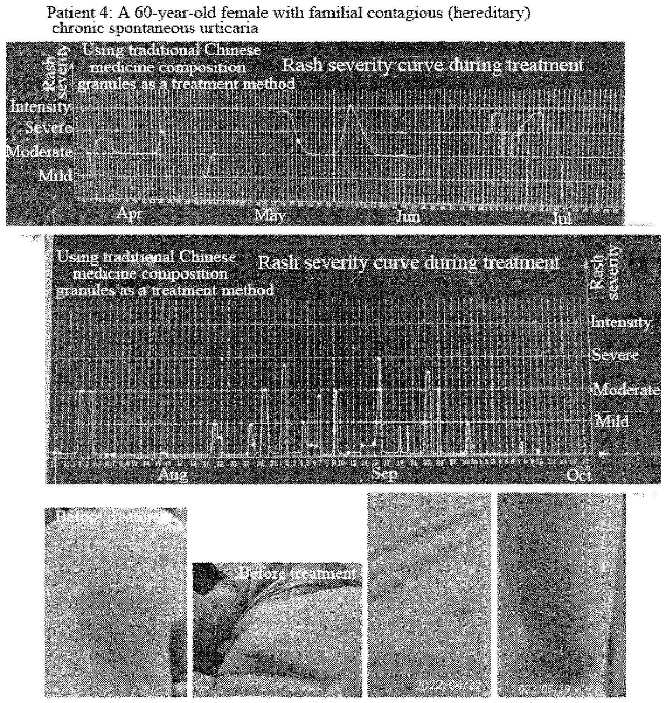

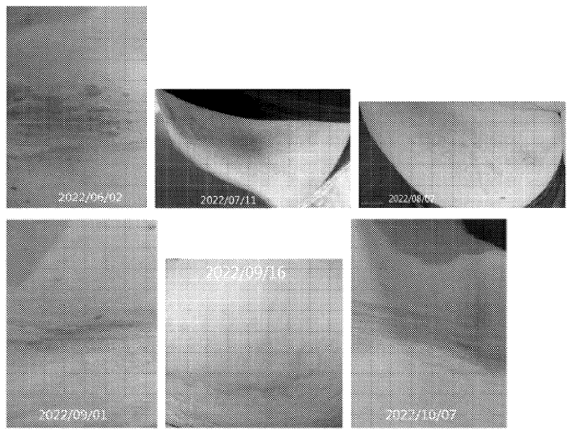

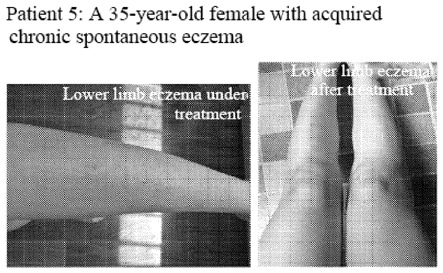

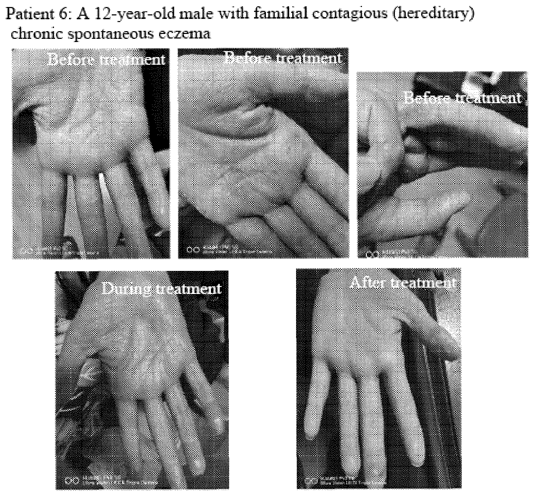

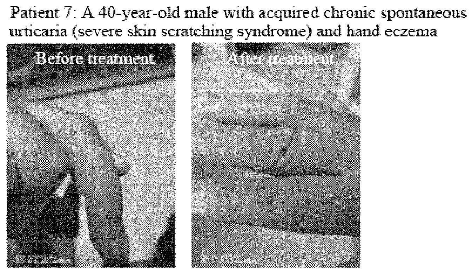

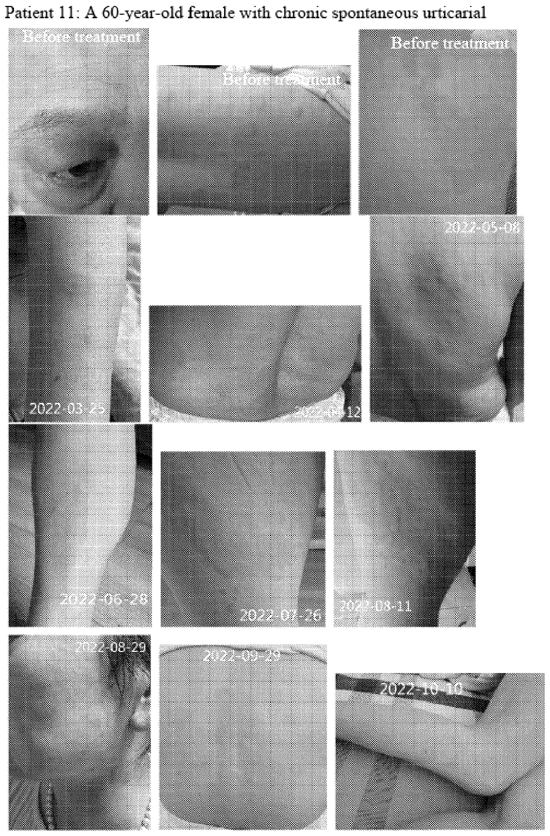

The healing process of typical skin rashes was shown in Patient 4 of the drawing in the specification.

### Patient 5: A 35-year-old female with acquired chronic spontaneous eczema

On August 27, 2021, the patient went to the Department of Traditional Chinese Medicine, Bengbu Hospital Of Traditional Chinese Medicine, for treatment due to severe eczema on both legs. Every year at the beginning of summer, the patient's chronic eczema broke out spontaneously on both legs and was covered with papules. The rash around the papules was red and irregular in shape with severe itching, and it was especially severe at night and led to particularly poor sleep quality. In autumn, the eczema subsided on its own. The patient had this condition since she was pregnant and gave birth 9 years ago. In the past, the patient took traditional Chinese medicine decoction orally to treat chronic eczema attacks, and the effect of controlling itching was obvious. However, this prescription used put a serious burden in the digestive system and caused severe diarrhea. And the effect of this prescription in controlling itching gradually became worse later. The dialectical prescription of the Department of Traditional Chinese Medicine was: 8 g of bran-fried *Rhizoma Atractylodis,* 10 g of *Cortex Phellodendri,* 10 g of ginger-processed *Caulis Bambusae in Taeniam,* 8 g of ginger-processed *Magnoliae officinalis cortex,* 10 g of *Semen Coicis,* 15 g of stir-fried rice sprout, 15 g of stir-fried *Fructus Hordei Germinatus,* 8 g of bran-fried *Fructus Aurantii,* 10 g of *Fructus Kochiae,* 10 g of *Herba Sedi,* 8 g of *Herba Eupatorii,* and 8 g of corn stigma, a total of 7 doses with 12 Chinese herbal medicines, 1 dose per day, half in the morning and half in the evening, by decoction in water. After the patient took the prescription orally, the eczema on legs still itched severely and she could not sleep at night, and the medication stopped after 3 doses of the medicine.

Starting from August 31st, the patient was given oral administration + external application of the traditional Chinese medicine composition I used by Patient 1 (granules): excluding *Salviae miltiorrhizae radix et rhizoma, Isatidis folium,* and *Radix Angelicae* , and the ingredients of traditional Chinese medicine composition II were added: 6 g of *Chuanxiong rhizoma,* 10 g of *Flos Lonicerae* , and 10 g of *Taraxaci herba,* 1 dose per day, half in the morning and half at night, with 300 mL of warm water. After taking the traditional Chinese medicine composition I (adjusted prescription) internally, the severe itching of eczema on the legs was quickly relieved. However, external application of the traditional Chinese medicine composition I (adjusted prescription) to the rash on the legs might cause the itching of the rash to worsen in a short period of time. (Analysis: the external application of traditional Chinese medicine liquid to the rash could stimulate the rash pathogens, intensify the activity of pathogens that were not inhibited and killed, thus causing the rash to become itchier in a short period of time.) After taking the medicine for 4 doses, the patient's symptoms of eczema on both legs were significantly relieved, the severe itching became mild, and the rash on both legs and the skin scratched during the itching became scabs. Later, the patient developed diarrhea when taking the traditional Chinese medicine composition I. Since the diarrhea was not serious, the patient was advised to continue taking the medicine. Since oral administration of this traditional Chinese medicine composition could intensify the detoxification of the patient's body, a certain degree of diarrhea was beneficial to the rapid cure of the patient's eczema. Therefore, the patient's medication status was not changed, and she persisted in taking the medication for more than two months. In the summer of 2022, the eczema rash on legs and other parts of the body no longer occurred, and the severe chronic spontaneous eczema that had lasted for more than 9 years was cured.

The healing process of typical skin rashes was shown in Patient 5 of the drawing in the specification.

### Patient 6: A 12-year-old male with familial contagious (hereditary) chronic spontaneous eczema

The patient's father once suffered from severe hand eczema, with cracking and peeling skin, rashes, and severe itching. Later, the symptoms of hand eczema subsided, and then he developed systemic itching, especially severe itching in the back, inner thigh muscles, and scrotum. The patient had been suffering from chronic spontaneous eczema since birth. The eczema on the hands was severely peeling, cracking and bleeding, the rash was severe, and itching was severe in summer; urticaria-like papules appeared on the forehead, and persisted throughout the summer. Starting from October 5, 2022, this patient tried the mixed adjusted prescription of compositions I and II (traditional Chinese medicine granule formula): 6.3 g of *Coptidis Rhizoma,* 10 g of *Scutellariae Radix,* 10 g of *Forsythiae fructus,* 10 g of *Perillae folium,* 10 g of *Trichosanthis radix,* 10 g of *Belamcandae rhizoma,* 9 g of *Glycyrrhizae radix et rhizoma,* 9 g of honey-processed *Stemonae radix,* 10 g of *Flos Lonicerae* , 12 g of *Herba Menthae,* 10 g of *Paeoniae alba radix,* 10 g of *Taraxaci herba,* 3 g of bran-fried *Atractylodis macrocephalae rhizoma,* 10 g of salt-processed *Anemarrhenae rhizoma,* 10 g of *Violae herba,* 6 g of *Chuanxiong rhizoma,* 10 g of *Schizonepetae herba,* 10 g of *Flos Dendranthema morifolium,* and 10 g of *Corni fructus,* a total of 7 doses with 19 components. The medication was 1 dose per day, half in the morning and half in the evening, taken orally with 100 mL of warm water. When taking orally, a small amount of traditional Chinese medicine liquid was left to apply externally to the eczema areas of the hands. After taking 3 doses of traditional Chinese medicine granules internally and externally on October 9, the urticaria-like papules on the forehead that had lasted for half a year began to subside significantly, the severe hand eczema stopped peeling, the rash gradually disappeared, and the symptoms of hand eczema and forehead urticaria-like papules improved rapidly and at a speed visible to the naked eye. On October 14, after taking 7 doses of the traditional Chinese medicine compositions for oral administration and external application, the symptoms of peeling and red rashes of hand eczema all subsided, and the skin on the hands completely returned to a healthy state. In order to consolidate the curative effect and completely eradicate the pathogen, the patient continued to take the traditional Chinese medicine granule composition orally.

The healing process of typical skin rashes was shown in Patient 6 of the drawing in the specification.

### Patient 7: A 40-year-old male with acquired chronic spontaneous urticaria (severe skin scratching syndrome) and hand eczema

On May 16, 2020, the patient reported that he had suffered from urticaria for more than 1 year, which occurred without triggers. During the attack, he had severe itching in the muscles of his inner thigh. The patient did not dare to rub the skin on the inner thigh when taking a bath, otherwise erythema and severe itching would appear immediately at the friction site. The patient's itching worsened after taking a bath, accompanied by erythema, and the epidermis became ulcerated and blood-red after scratching. The patient took the cross-adjusted granules of traditional Chinese medicine composition I and traditional Chinese medicine composition II orally. A total of 5 doses of the traditional Chinese medicine composition I (adjusted ingredients) was applied, 1 dose per day, fully dissolved in about 300 mL of warm water, half dose in the morning and half dose in the evening, or taken orally three times in the morning, noon, and evening. At the same time, an aqueous solution of the composition was applied to areas with severe itching of the skin. After taking the first dose orally, the patient's itching in the inner thigh muscles seemed to worsen, but the nerves throughout his body were relaxed and calm, he felt sleepy and had a high quality sleep that night. After the second dose, the symptoms of itching in the inner thigh muscles were greatly improved, and the habitual itching after bathing was no longer present. The symptoms disappeared after 5 doses, and the inner thigh muscles no longer itched after bathing. Then the patient took the traditional Chinese medicine composition II (adjusted ingredients) orally, 3 doses in total, 1 dose per day, fully dissolved in about 300 mL of warm water, half dose in the morning and evening, or taken three times in the morning, noon and evening. From the beginning of taking the traditional Chinese medicine composition I orally, and then taking the traditional Chinese medicine composition II, the patient could feel sleepy all day long, and the drug showed an extremely strong sedative effect, indicating that his body's nervous system was completely relaxed. The itching of the skin no longer occurred when rubbing skin during the shower.

On September 11, 2020, the patient consulted again and said that because he had to wear plastic protective gloves at work, long-term work cause pimples the size of rice grains on his fingers of both hands with severe itching. When the pimples were pricked, fluid would flow out, and his fingers were swollen and painful, partially cracked, and the skin was dry and peeling. Judging from the symptoms, it was eczema on the hands. The pathogenesis of eczema and urticaria was similar, the root causes of both were infection by microbial pathogens in the body. So the patient was given traditional Chinese medicine composition I (granules) for oral administration, 10 doses in total, 1 dose per day, fully dissolved in about 300 ml of warm water, half dose in the morning and half in the evening. When the skin itches was severe, the medicine was applied externally at the same time. After taking 3 doses orally, the swelling of fingers was relieved, skin cracking was reduced, and itching was reduced. After the 4th to 5th dose, the patient developed mild diarrhea. At the same time, the swelling and pain in the hands and fingers disappeared, the itching disappeared, the skin cracks were slowly healing, and the dryness of the skin was relieved, but the peeling still occurred. On the 7th to 8th dose, after taking the medicine, the patient experienced mild stomach discomfort, acid reflux and bitter taste in mouth. Except for the dry and peeling skin on hands, other symptoms disappeared.

The healing process of typical skin rashes was shown in Patient 7 of the drawing in the specification.

### Patient 8: A 37-year-old male with acute urticaria

In August 2020, the patient reported that he went to the rice field to spray pesticides the day before. After returning home, pimples of different sizes began to appear on the right side of his waist. The pimples were connected into one piece. The pimples were surrounded by bright red allergic skin, which caused severe itching and was very unbearable. (The patient had allergic constitution and reported that his back generally itched when sleeping at night)

Some of the components in traditional Chinese medicine composition I and traditional Chinese medicine composition II were selected: 10 g of *Trichosanthis radix,* 12 g of *Radix Angelicae* , 10 g of *Taraxaci herba,* 10 g of *Scutellariae Radix,* 10 g of *Paeoniae alba radix,* 10 g of *Cortex Phellodendri,* 6 g of *Ramulus Cinnamomi,* 10 g of *Forsythiae fructus,* 10 g of salt-processed *Anemarrhenae rhizoma,* 9 g of *Glycyrrhizae radix et rhizoma,* 10 g of stir-fried *Cassiae semen,* and 10 g of *Atractylodis macrocephalae rhizoma,* a total of 12 components. The prescription (traditional Chinese medicine granules) was 4 doses, which should be taken internally and applied externally to the rash. The medication was to be taken orally twice a day, 1 dose per day, fully dissolved in 200 mL of warm water, half dose in the morning and evening, and applied externally to the rash at least 3 to 4 times a day. After taking one dose of the medicine orally and applying a small amount of the medicine externally to the rash, the severe itching symptoms disappeared, and the bright red skin around the allergic pimples and edema turned into mild itching. After taking this prescription internally and externally, the symptoms gradually eased. After 4 doses of medicine, the rash and edema disappeared, the itching completely disappeared, and the bright red skin due to allergies turned into red pimples the size of mung beans. This patient thought the medicine was too bitter and stopped taking, and then the allergic symptoms gradually disappeared on their own.

### Patient 9: A 19-year-old male with acquired chronic spontaneous urticaria

This patient had severe itching on his back, inner thighs, scrotum, and anus all year round, with unexplained attacks and widespread and irregular attacks in other parts. Before and during weather changes, the itching was particularly severe, and the itching was also severe at night. The disease lasted for many years. None of his parents and other immediate family members had allergic diseases.

Starting from April 12, 2020, the patient took the traditional Chinese medicine composition 3 (traditional Chinese medicine granules) orally (taking part of the ingredients of the traditional Chinese medicine composition I): 12 g of ginger-processed *Magnoliae officinalis cortex,* 12 g of *Radix Angelicae* , 10 g of *Atractylodis macrocephalae rhizoma,* 10 g of *Scutellariae Radix,* 10 g of *Paeoniae alba radix,* 10 g of *Radix Astragali,* 6 g of *Coptidis Rhizoma,* 6 g of *Ramulus Cinnamomi,* 10 g of *Forsythiae fructus,* 10 g of salt-processed *Anemarrhenae rhizoma,* 9 g of *Glycyrrhizae radix et rhizoma,* and 10 g of stir-fried *Cassiae semen,* a total of 15 doses with 12 components, 1 dose per day, fully dissolved in 200 mL warm water, half dose in the morning and half in the evening.

Then the patient took traditional Chinese medicine composition 4 (traditional Chinese medicine granules) orally (taking part of the ingredients in traditional Chinese medicine composition I and composition II): 10 g of *Taraxaci herba,* 10 g of *Atractylodis macrocephalae rhizoma,* 10 g of *Scutellariae Radix,* 10 g of *Paeoniae alba radix,* 10 g of *Radix Astragali,* 6 g of *Ramulus Cinnamomi,* 10 g of *Forsythiae fructus,* 10 g of salt-processed *Anemarrhenae rhizoma,* 9 g of *Glycyrrhizae radix et rhizoma,* 10 g of *Trichosanthis radix,* 6 g of *Rhei radix et rhizoma,* and 6 g of *Radix Angelicae* , a total of 20 doses with 12 components, the oral administration method was the same as that in composition 3. Diarrhea occurred after oral administration (the prescription included 6 g of *Rhei radix et rhizoma* for detoxification).

In addition, the patient had previously taken Xueduwan Pills for Skin Diseases from Tongrentang orally to treat chronic urticaria symptoms. After comparing the compositions 3 and 4 with the Xueduwan Pills for Skin Diseases from Tongrentang, it was found that the three medicines showed similar effects, and the itching symptoms were relieved after taking the medicine.

In February 2021, the patient took traditional Chinese medicine composition I and traditional Chinese medicine composition II granules orally.

The patient reported that traditional Chinese medicine composition I and traditional Chinese medicine composition II were more effective than compositions 3, 4, and Xueduwan Pills for Skin Diseases, and had better antipruritic effects, but they were not as desirable at controlling itching as ebastine. After taking ebastine orally, the allergic symptoms were immediately controlled and the itching completely disappeared. However, the urticaria would recur on its own soon after, with the same symptoms as before, without any relief. The patient insisted on taking ebastine orally for more than 6 months. During the treatment period, he took ebastine when symptoms of allergies and itching appeared, and stopped taking the medicine when the symptoms disappeared. After more than 6 months of treatment, the frequency of chronic spontaneous urticaria attacks had not decreased, and the wheal rash and itching had not been alleviated.

### Patient 10: A 31-year-old male with chronic spontaneous urticaria complicated by digestive system diseases

On January 14, 2021, the patient, an emergency physician, reported that he had been suffering from urticaria for many years and that it broke out spontaneously without obvious triggers. When urticaria recurred, wheals and bumps might appear on the arms and shoulders, and bright red wheals and rashes could appear on the back and inner thighs. Allergic wheals, bumps and rashes in other parts of the body might be widespread and had no fixed location, and the itching was unbearable and recurred all year round. The patient had diarrhea symptoms all year round, accompanied by a dull pain in the gastrointestinal tract. The discomfort was particularly prominent and recurred at wee hours of everyday (gastrointestinal disease was also a common complication of the patients with chronic urticaria). Before using traditional Chinese medicine composition to treat the disease, he tried a variety of external ointments, but none of them could relieve the itching. The patient was recommended to try three traditional Chinese medicine compositions to control symptoms of chronic urticaria.

Chinese patent medicine Xunmazhenwan Pills for urticaria, 1 bag at one time, taken orally once in the morning and evening separately. After taking the medicine, the patient experienced acid reflux, abdominal pain, and diarrhea, and was unable to control severe itching of his skin at night. He stopped taking the medicine after 2 d.

The traditional Chinese medicine composition I (granules with adjusted ingredients) : removing stir-fried *Atractylodis macrocephalae rhizoma, Rhizoma Dryopteris Crassirhizomae, Scutellariae Radix,* and *Forsythiae fructus,* and adding *Flos Lonicerae* , was used 5 doses in total, 1 dose per day, fully dissolved in about 300 mL of warm water, half dose in the morning and evening separately. After half a dose of this prescription was taken orally, and applied externally to the areas with severe itching, the nervous system of the whole body could soon be relaxed, the skin could feel comfortable and relaxed, the nerve endings of the skin could be relaxed and sensitive, the itching all over the body was completely controlled, the severely itchy parts no longer itched when sleeping at night, the patient felt relaxed with greatly improved sleep quality. After further oral administration of this formula, the diarrhea was relieved and the gastrointestinal discomfort in the early morning disappeared.

The traditional Chinese medicine composition II (cross-adjusted granules with the ingredients of composition I) : removing *Flos Caryophylli, Corni fructus, Flos Lonicerae , Zingiberis rhizoma recens, Rhei radix et rhizoma,* and *Senecionis scandentis herba,* and adding *Scutellariae Radix, Forsythiae fructus,* and stir-fried *Atractylodis macrocephalae rhizoma,* was used 5 doses in total, 1 dose per day, fully dissolved in about 300 mL of warm water, half dose in the morning and evening separately. This composition could not completely relieve itching, but drove the source of infection in the body to the skin. After taking the first 3 doses, the itching of the skin on the arms, shoulders, and inner thighs intensified, while severe angioedema appeared, and large connected bumps appeared in several places on the arms and shoulders. Meanwhile, the traditional Chinese medicine composition II was applied externally in liquid form, and the itching was relieved. Although the skin allergy symptoms worsened after taking the medicine, the patient felt that it had a positive therapeutic effect, and the whole nervous system became relaxed and no longer catatonic excitement.

### Patient 11: A 60-year-old female with chronic spontaneous urticaria

The patient had chronic spontaneous urticaria for many years, with widespread erythema all over the body, and angioedema bags of varying density and size appearing on the upper limbs, head, neck, and knee pits, with different skin colors; severe itching occurred, especially at night, and sleep quality was poor. During more than two years of treatment, the patient had been taking the antihistamine chlorpheniramine maleate tablets and the hormone dexamethasone tablets orally. After taking the hormones orally, the itching relieving effect was desirable, but the disease relapsed when the medication was stopped, and there was swelling of the face, eyes, and limbs, which could not be healed after long-term treatment. The patient had tried traditional Chinese medicine composition decoction for treatment, but the effect of relieving itching was poor.

In March 2022, the patient consulted Patient 1 on the recommendation of a friend. Since this patient was deaf-mute, her friend reported that the patient's itching symptoms were not serious, and the patient's rash symptoms were not severe at the time. Therefore, the recommended traditional Chinese medicine composition (traditional Chinese medicine granules) was: 9 g of *Glycyrrhizae radix et rhizoma,* 12 g of *Herba Menthae,* 10 g of bran-fried *Atractylodis macrocephalae rhizoma,* 10 g of *Moslae herba,* 10 g of *Taraxaci herba,* 10 g of *Belamcandae rhizoma,* 10 g of *Flos Lonicerae* , 15 g of *Caulis Lonicerae,* 12 g of *Ramulus Cinnamomi,* 10 g of stir-fried *Fructus Arctii,* 10 g of *Moutan cortex,* 10 g of *Dictamni cortex,* 10 g of *Folium Mori,* 10 g of *Flos Dendranthema morifolium,* 10 g of *Magnoliae flos,* 10 g of *Radix Astragali,* 6 g of honey-processed *Herba Ephedrae,* 10 g of *Schizonepetae herba,* and 6 g of *Radix Sophorae Flavescentis,* a total of 5 doses with 19 components. The medication was given 1 dose per day, 300 mL warm boiled water for oral administration + external application to the skin with severe itching, half dose in the morning and evening separately or 1 dose three times in the morning, noon and evening. After taking this prescription orally, the patient's skin rash suddenly worsened, with erythema and rash accompanied by angioedema, intensified itching, and generalized rash all over the body. Although the external application of the traditional Chinese medicine solution could slightly relieve the skin itching, the skin itching was still severe, and the patient felt cold after the 4th and 5th dose. (Analysis: the patient had chronic urticaria for many years, and her conditions were actually serious. This prescription could not completely control the patient's symptoms, but had a certain therapeutic effect; instead, the prescription drove the pathogens in the patient's body to the surface skin, causing the symptoms of intensified rash and itching; the rash was accompanied by skin angioedema, causing the patient to show symptoms of chills.) This prescription was stopped after medication.

The patient was then given traditional Chinese medicine composition I granules, which were taken orally and applied externally to the severe itching areas of the skin. When the itching occurred all over the body, the patient was given oral antihistamines and the hormone dexamethasone tablets. After taking traditional Chinese medicine composition I orally, although the patient's rash symptoms were still severe, the severe itching was relieved, her sleep improved at night, and she felt a warm feeling in her body after taking the medicine. After 7 doses of this prescription, the erythema, rash and accompanying skin angioedema all subsided, the itching all over the body became less obvious, and the quality of sleep at night was greatly improved. After that, the patient continued to take traditional Chinese medicine composition I, traditional Chinese medicine composition II and a mixed adjusted prescription of the two compositions. During this period, the patient's rash recurred, and the itching persisted. During the rash, large areas of erythema generally appeared, and occasionally round or oval rashes with small protruding skin appeared. Occasionally, scattered papules appeared, and the symptoms were severe or even acute. When severe and acute skin rashes and erythema occurred, the patient took hormone dexamethasone tablets orally for anti-inflammation. The skin symptoms could subside quickly, but the skin symptoms might relapse the next day. When the patient did not take traditional Chinese medicine composition I or traditional Chinese medicine composition II orally before and only took hormones and antihistamines, although the patient's skin rash and erythema could be controlled, there might be accompanied by symptoms of general edema such as eye redness and swelling, facial swelling, and limb swelling. After taking the traditional Chinese medicine compositions I and II orally together with dexamethasone tablets and antihistamines as auxiliary drugs to control severe and intensity skin symptoms and itching, the patient's symptoms of general edema and eye swelling almost disappeared, the intensity of itching all over the body was greatly relieved, and the quality of sleep at night was also greatly improved. The patient's skin symptoms such as rash and erythema gradually worsened after starting treatment with traditional Chinese medicine compositions I and II (this situation was a manifestation of the efflux of dampness, heat and toxins from the body, and was in line with the regular characteristics of traditional Chinese medicine in the treatment of chronic urticaria), until the patient's rash and erythema reached their most severe state in July and August. Skin symptoms always ranged from severe to acute, but the severe itching all over the patient's body was gradually relieved. It was not until September 20 that the patient's rash and erythema began to significantly reduce. By the end of September, the patient's skin symptoms were hovering at moderate or below levels. After October 10, the patient's skin symptoms such as rash and erythema became very mild, and the degree of itching also changed qualitatively. The patient still insisted on continuing to take traditional Chinese medicine composition I, traditional Chinese medicine composition II and a mixture of the two compositions to adjust the formula, in order to completely cure chronic spontaneous urticaria.

The typical skin rash healing process was shown in Patient 11 in the drawings of the specification.

### Patient 12: A 12-year-old male with allergic rhinitis complicated by allergic conjunctivitis

The patient sneezed early in the morning, almost every day, and his eyes always itched, manifesting as chronic conjunctivitis. The syndrome was diagnosed as allergic rhinitis and conjunctivitis. Starting from August 8, 2022, the 18-component traditional Chinese medicine granule composition was taken orally, which was prepared by mixed adjustment of traditional Chinese medicine composition I and traditional Chinese medicine composition II. After taking the composition orally for 8 doses, the patient felt that the number of sneezes in the morning was slightly reduced and nasal congestion began to occur. The symptoms of chronic conjunctivitis and eye discomfort still existed in the eyes. The frequency of urination increased after taking the medicine, and there was no diarrhea or stomach discomfort. After taking 15 doses orally, the frequency of sneezing in the morning was obviously slightly reduced, and occasional mild runny nose appeared after nasal congestion. After taking 35 doses orally, the patient intuitively felt that the symptoms had changed significantly: the nasal congestion was no longer severe when sleeping at night. The patient then took a prescription of 19-component traditional Chinese medicine granules, which was prepared by mixed adjustment of traditional Chinese medicine compositions I and II, for a total of 10 doses. After taking 45 doses, a 20-component traditional Chinese medicine granule composition was taken orally, which was prepared by mixed adjustment of traditional Chinese medicine composition I and traditional Chinese medicine composition II. After taking 50 doses of traditional Chinese medicine granules orally, the number of days without sneezing in the morning increased from 2-3 days to 4-5 consecutive days, and allergic rhinitis was significantly improved; symptoms of nasal congestion and runny nose were also significantly reduced; the itching sensation of chronic conjunctivitis of the eyes also became mild. In order to consolidate the previous curative effect and cure rhinitis and conjunctivitis caused by allergic constitution, the patient insisted on continuing to take the 20-component traditional Chinese medicine granule composition prepared by mixed adjustment of traditional Chinese medicine compositions I and II.

## Claims

1. A traditional Chinese medicine composition I, prepared from scientific extracts from 20 Chinese herbal medicines of *Scutellariae Radix, Coptidis Rhizoma, Forsythiae fructus, Perillae folium, Magnoliae officinalis cortex, Atractylodis macrocephalae rhizoma, Radix Angelicae , Paeoniae alba radix, Cassiae semen, Cortex Phellodendri, Stemonae radix, Artemisiae annuae herba, Anemarrhenae rhizoma, Trichosanthis radix, Glycyrrhizae radix et rhizoma, Herba Menthae, Rhizoma Dryopteris Crassirhizomae, Salviae miltiorrhizae radix et rhizoma, Violae herba,* and *Isatidis folium*; wherein the 20 Chinese herbal medicines have the following contents: 9 g to 10 g of the *Scutellariae Radix,* 6 g to 8 g of the *Coptidis Rhizoma,* 10 g to 12 g of the *Forsythiae fructus,* 10 g of the *Perillae folium,* 10 g to 12 g of ginger-processed *Magnoliae officinalis cortex,* 10 g of stir-fried *Atractylodis macrocephalae rhizoma,* 10 g to 12 g of the *Radix Angelicae* , 10 g to 12 g of the *Paeoniae alba radix,* 10 g to 15 g of stir-fried *Cassiae semen,* 9 g to 12 g of the *Cortex Phellodendri,* 10 g of the *Stemonae radix,* 10 g to 12 g of the *Artemisiae annuae herba,* 10 g to 12 g of salt-processed *Anemarrhenae rhizoma,* 10 g to 12 g of the *Trichosanthis radix,* 9 g to 10 g of the *Glycyrrhizae radix et rhizoma,* 6 g to 12 g of the *Herba Menthae,* 10 g to 12 g of the *Rhizoma Dryopteris Crassirhizomae,* 8 g to 10 g of the *Salviae miltiorrhizae radix et rhizoma,* 10 g to 15 g of the *Violae herba,* and 10 g to 15 g of the *Isatidis folium*; and the traditional Chinese medicine composition I is made using the scientific extracts of the 20 components of Chinese herbal medicines, and then prepared into a medicine for oral administration comprising an oral pill, a powder, and a capsule; and the scientific extracts of the 20 components are also able to be prepared into a medicine for external application and topical application comprising a medicine liquid for external application, an ointment, or a patch that is used for external application and topical application in conjunction with the medicine for oral administration in areas with severe itching of the skin.

2. A traditional Chinese medicine composition II, prepared from scientific extracts from 21 Chinese herbal medicines of *Glycyrrhizae radix et rhizoma, Herba Menthae, Chuanxiong rhizoma, Gentianae radix et rhizoma, Moslae herba, Taraxaci herba, Rhei radix et rhizoma, Senecionis scandentis herba, Zingiberis rhizoma recens, Flos Lonicerae* , *Chebulae fructus, Belamcandae rhizoma, Pulsatillae radix, Moutan cortex, Magnoliae flos, Corni fructus, Herba Agastachis, Picrorhizae rhizoma, Dictamni cortex, Schizonepetae herba,* and *Flos Dendranthema morifolium*; wherein the 21 Chinese herbal medicines have the following contents: 9 g to 10 g of the *Glycyrrhizae radix et rhizoma,* 6 g to 12 g of the *Herba Menthae,* 6 g to 10 g of the *Chuanxiong rhizoma,* 5 g to 6 g of the *Gentianae radix et rhizoma,* 15 g to 20 g of the *Senecionis scandentis herba,* 15 g to 20 g of the *Pulsatillae radix,* 9 g to 10 g of the *Moslae herba,* 10 g to 15 g of the *Taraxaci herba,* 10 g to 15 g of the *Flos Lonicerae* , 9 g to 10 g of the *Belamcandae rhizoma,* 9 g to 10 g of the *Moutan cortex,* 9 g to 10 g of the *Magnoliae flos,* 5 g to 6 g of prepared *Rhei radix et rhizoma,* 9 g to 10 g of the *Zingiberis rhizoma recens,* 6 g to 10 g of the *Chebulae fructus,* 10 g to 12 g of the *Dictamni cortex,* 9 g to 10 g of the *Cornifructus,* 10 g to 12 g of the *Flos Dendranthema morifolium,* 9 g to 10 g of the *Schizonepetae herba,* 3 g of the *Picrorhizae rhizoma,* and 9 g to 10 g of the *Herba Agastachis*; and the traditional Chinese medicine composition II is made using the scientific extracts of the 21 Chinese herbal medicines, and then prepared into a medicine for oral administration comprising an oral pill, a powder, and a capsule; and the scientific extracts of the 21 Chinese herbal medicines are also able to be prepared into a medicine for external application and topical application comprising a medicine liquid for external application, an ointment, or a patch that is used for external application and topical application in conjunction with the medicine for oral administration in areas with severe itching of the skin.

3. A traditional Chinese medicine compositions with 16 to 21 components, wherein the traditional Chinese medicine compositions are prepared from 39 components in the traditional Chinese medicine composition I and the traditional Chinese medicine composition II, and each of the components are weighed according to the contents of the corresponding components in claims 1 and 2; and the components are scientifically extracted and then combined into the traditional Chinese medicine compositions to be further prepared into a medicine for oral administration or a medicine for external use.

4. According to claims 1, 2, and 3, extracting the active ingredients of the traditional Chinese medicine compositions or some components of the traditional Chinese medicine compositions to prepare descriptions, forming a dosage comprising an oral pill, a powder, a granule, a tablet, and a capsule; alternatively, the active ingredients of the descriptions are extracted to form an injection; alternatively, the active ingredients of the traditional Chinese medicine description or some of the components are extracted to form a medicine for external use comprising an ointment, a medicine liquid for external application, and a patch, or a medicinal bath product; all above fall with the claimed scope of the patent; the invention treats various types of acute and chronic urticaria, acute and chronic eczema, allergic constitutions, and also treats allergic conjunctivitis, chronic pharyngitis, allergic rhinitis/sinusitis, allergic bronchitis/asthma, oral malignant boils and pyogenic infections, sticky and thick tongue coating, severe bad breath, chronic (ulcerative) gastroenteritis, colitis, proctitis, concurrent joint pain and muscle pain, physical weakness, concurrent ear canal itching, pruritus vulvae, scrotal itching, anal pain and itching, hot eyes and itchy eyes; the traditional Chinese medicine composition further treats complications of poor sleep quality, biological clock/endocrine disorders, cranial neurasthenia, complications of listlessness, hyperactivity, and alternating occurrence of the listlessness and the mental excitation, influenza virus, scabies mites, *Helicobacter pylori, Mycoplasma pneumoniae, Shigella dysenteriae,* various types of ringworm and other microbial infections.
